(19) Europäisches Patentamt
European Patent Office
Office européen des brevets

(11) Publication number: 0 260 588 B1

# (12) EUROPEAN PATENT SPECIFICATION

(45) Date of publication of the patent specification: 01.08.90

(51) Int. Cl.⁵: C07D 473/30

(21) Application number: 87113168.6

(22) Date of filing: 09.09.87

## (54) Process for the preparation of alpha-N-[(hypoxanthin-9-yl)-pentyloxycarbonyl]-arginine.

(30) Priority: 17.09.86 IT 2173486

(43) Date of publication of application: 23.03.88 Bulletin 88/12

(45) Publication of the grant of the patent: 01.08.90 Bulletin 90/31

(84) Designated Contracting States: AT BE CH DE ES FR GB GR LI LU NL SE

(56) References cited:
EP-A- 0 009 155
EP-A- 0 077 460

(73) Proprietor: Sigma-Tau Industrie Farmaceutiche Riunite S.p.A., 47, Viale Shakespeare, I-00144 Rome(IT)

(72) Inventor: Stradi, Riccardo, Via Sansovino, 33, I-20133 Milano(IT)

(74) Representative: Bianchetti, Giuseppe, Studio Consulenza Brevettuale Via Rossini, 8, I-20122 Milan(IT)

EP 0 260 588 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

## Description

The present invention refers to a new and improved process for the preparation of α-N-[(hypoxanthin-9-yl-pentyloxycarbonyl]-arginine, of formula I

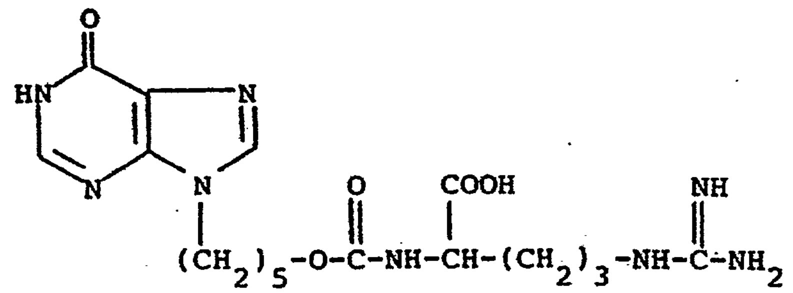

(I)

Compound I, characterized by marked immunomodulating activity, is claimed - with other derivatives - in the European patent application No. 77460, and it was up to now prepared by direct acylation of arginine with (hypoxanthin-9-yl)pentyloxycarbonylchloride.

The compound so obtained - in low yields - was however mixed with impurities of different kinds, mainly comprising the isomer N-[(hypoxanthin-9-yl)-pentyloxycarbonyl]-N′-[(4-amino-4-carboxy)butyl]-arginine II:

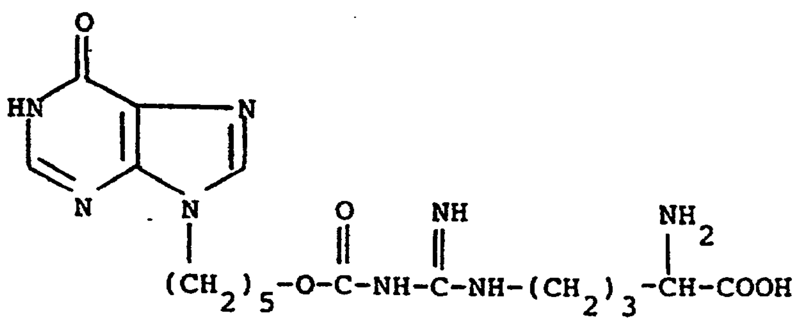

(II)

The purification of I, particularly from II, is moreover particularly difficult.

It has now been found that compound I, free from impurities, including isomer II, may be prepared in good yields by acylating $N_7$-nitro-arginine IV with (hypoxanthin-9-yl)pentyloxycarbonylchloride III and eliminating thereafter, by means of hydrogenolysis on palladium on charcoal, the nitro group from the intermediate V so obtained. The overall scheme of the new process may be so illustrated.

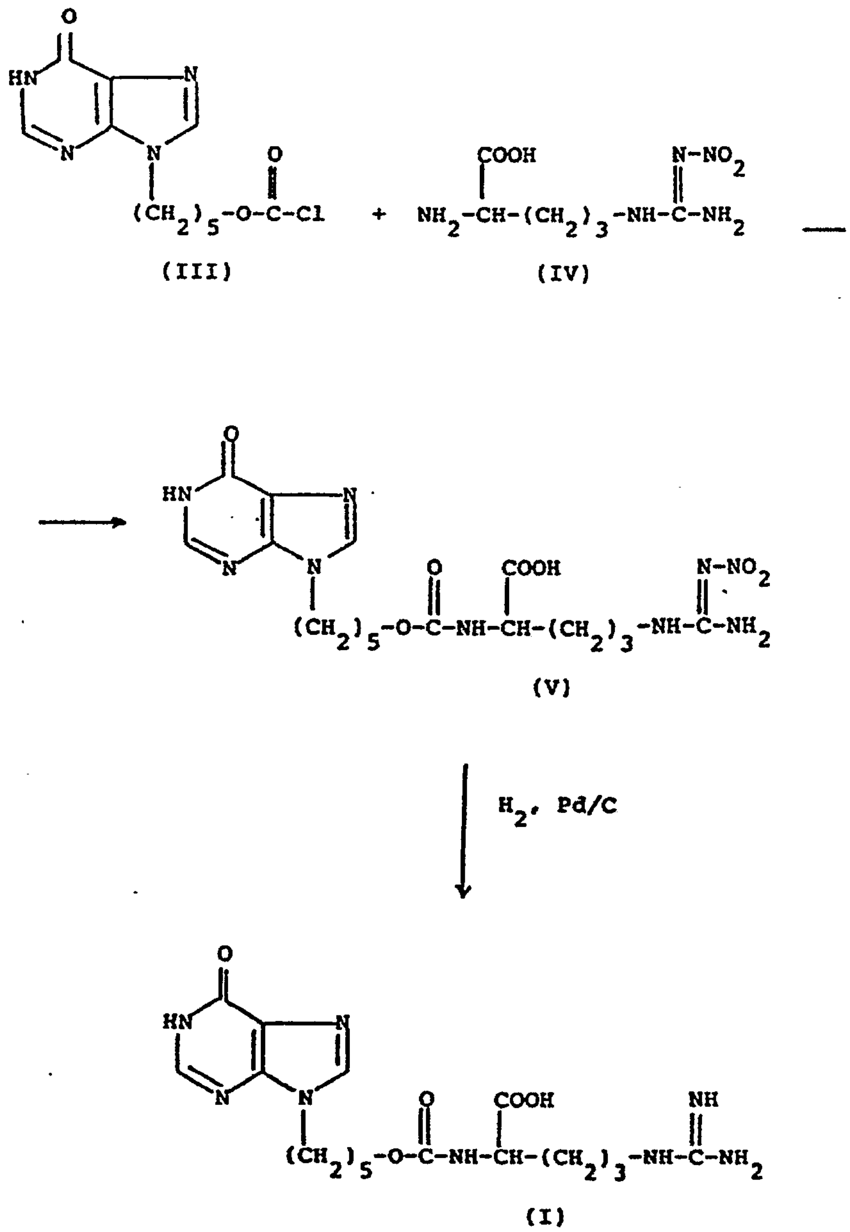


(III) (IV)

(V)

(I)

The chlorocarbonate III is obtained by treating with phosgene in an inert solvent from the corresponding alcohol 5-(hypoxanthin-9-yl)pentanol-1, already disclosed in the European patent application 77460; halohydrocarbons or arenes, namely toluene, are particularly suited as solvents.

The reaction is carried out at temperatures ranging from -10° to +30°C, preferably at a starting temperature of about 0-5°C, then at room temperature, conveniently with an excess of phosgene. After elimination of said excess at reduced pressure, the crude chlorocarbonate obtained is directly reacted with nitroarginine IV. Said reaction is preferably carried out in heterogeneous systems consisting of the suspension of III in an aprotic solvent (which may be the same in which compound III has been prepared) and from the aqueous solution of a nitroarginine salt, for instance the sodium or potassium salt; the reaction is carried out at temperatures ranging from 0 to 50°C, preferably at room temperature, under vigorous stirring.

The nitro derivative V is then subjected to hydrogenolysis on 5-10% palladium on charcoal, in alcoholic solution (methanol, ethanol, isopropanol) acidified with acetic acid.

The following example further illustrates the process of the invention, without limiting it in any way.

Although the example itself discloses the use of L-$N_7$-nitroarginine and, consequently, the obtainment of I wherein the arginine portion has L configuration, also the corresponding compounds with D or DL configuration are comprised in the scope of the invention.

EXAMPLE

a) (Hypoxanthin-9-yl)pentyloxycarbonylchloride (formula III)

A toluene solution (20% w/v) of $COCl_2$ (0.096 moles) was added dropwise, under stirring and cooling to 0-5° C, to a suspension of 18 g (0,081 moles) of 5-(hypoxanthin9-yl)-pentanol-1 in 500 ml of anhydrous toluene. When the addition was over, the temperature was allowed to rise to the room value and the mixture was stirred for 24 hours. The excess of phosgene and toluene were then eliminated under reduced pressure.

b) α-N-[(hypoxanthin-9-yl)-pentyloxycarbonyl]-$N_7$-nitroarginine (formula V)

The suspension of the crude chlorocarbonate obtained in a) in 150 ml of toluene was treated, under thorough stirring, with a solution of 17.64 g of $N_7$-nitroarginine (Formula IV) in 106 ml of 2N sodium hydroxide aqueous solution.
The mixture was stirred at room temperature for 12 hours, the phases were separated, the toluene layer discarded, the aqueous phase washed (which should have pH 4-5; if lower, NaOH should be added) with fresh toluene, then evaporated under vacuum.
The residue was purified by chromatography on silica gel; eluent ethylacetate: methanol in 1:1 ratio. 14 g of a product were obtained whose NMR, IR and mass (FD) spectra are in agreement with the structure V.

c) α-N-[(hypoxanthin-9-yl)-pentyloxycarbonyl]-arginine (formula I)

10 g of the intermediate V obtained in b) were dissolved in 200 ml of methanol + 30 ml of acetic acid; 2 g of 10% palladium on charcoal were added thereto and the mixture was hydrogenated at room pressure and temperature checking by HPLC the disappearance of the nitroderivative. The catalyst was eliminated by filtration and the mixture evaporated.
The product obtained with an almost quantitative yield is a crystalline hygroscopic solid, decomposing at 167-170°C in a sealed tube.

| Elemental analysis | | | |
|---|---|---|---|
| for $C_{17}H_{26}N_8O_5$ (MW = 422.4) | | | |
| calc. % | C = 48.34; | H = 6.20; | N = 26.52 |
| found % | C = 47.98; | H = 6.37; | N = 26.29. |

## Claims

1. A process for the preparation of α-N-[(hypoxanthin-9-yl)-pentyloxycarbonyl]-arginine of formula I, characterized in that α-N-[(hypoxanthin-9-yl)-pentyloxycarbonyl]-$N_7$-nitroarginine of formula V, obtained by acylation of $N_7$-nitroarginine IV with (hypoxanthin-9-yl)pentyloxycarbonyl chloride III, is subjected to hydrogenolysis according to the scheme

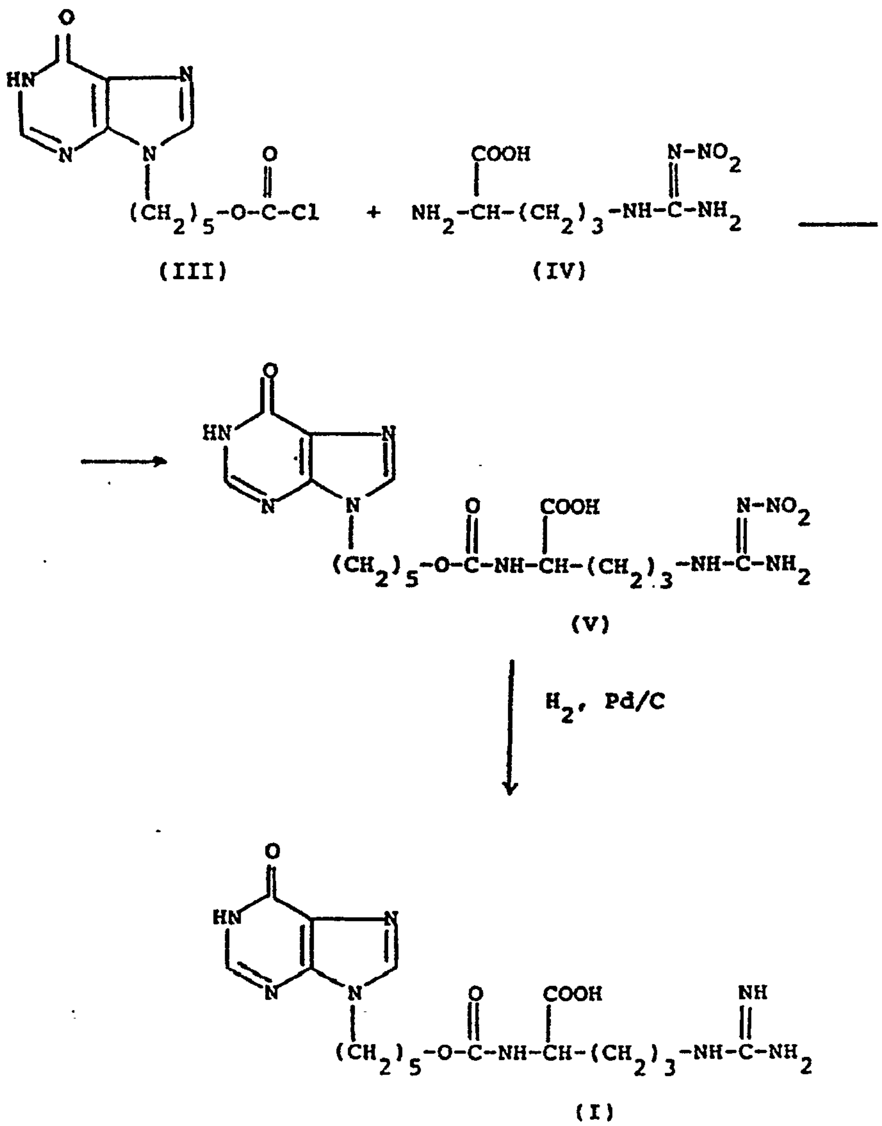


(III) (IV) (V) (I)

2. A process according to claim 1, characterized in that the hydrogenolysis of V is carried out on palladium on charcoal in alcoholic solution of acetic acid.

3. A process according to claim 2, characterized in that the reaction is carried out in methanol solution.

4. A process according to claim 1, characterized in that the acylation of IV with III is carried out in an heterogeneous system, consisting of an aqueous solution of a salt IV and of a suspension of III in an aprotic solvent.

5. A process according to claim 4, characterized in that the salt IV is the sodium or potassium salt

## Revendications

1. Un procédé de préparation de l'α–N-[(hypoxanthine-9-yl)-pentyloxycarbonyl]-arginine de formule I, caractérisé en ce que l'α-N-[(hypoxanthine-9-yl)-pentyloxycarbonyl]-$N_7$-nitroarginine de formule V, obtenue par acylation de $N_7$-nitroarginine IV avec du chlorure de (hypoxanthine-9-yl)-pentyloxycarbonyle III, est soumise à une hydrogénolyse selon le schéma:

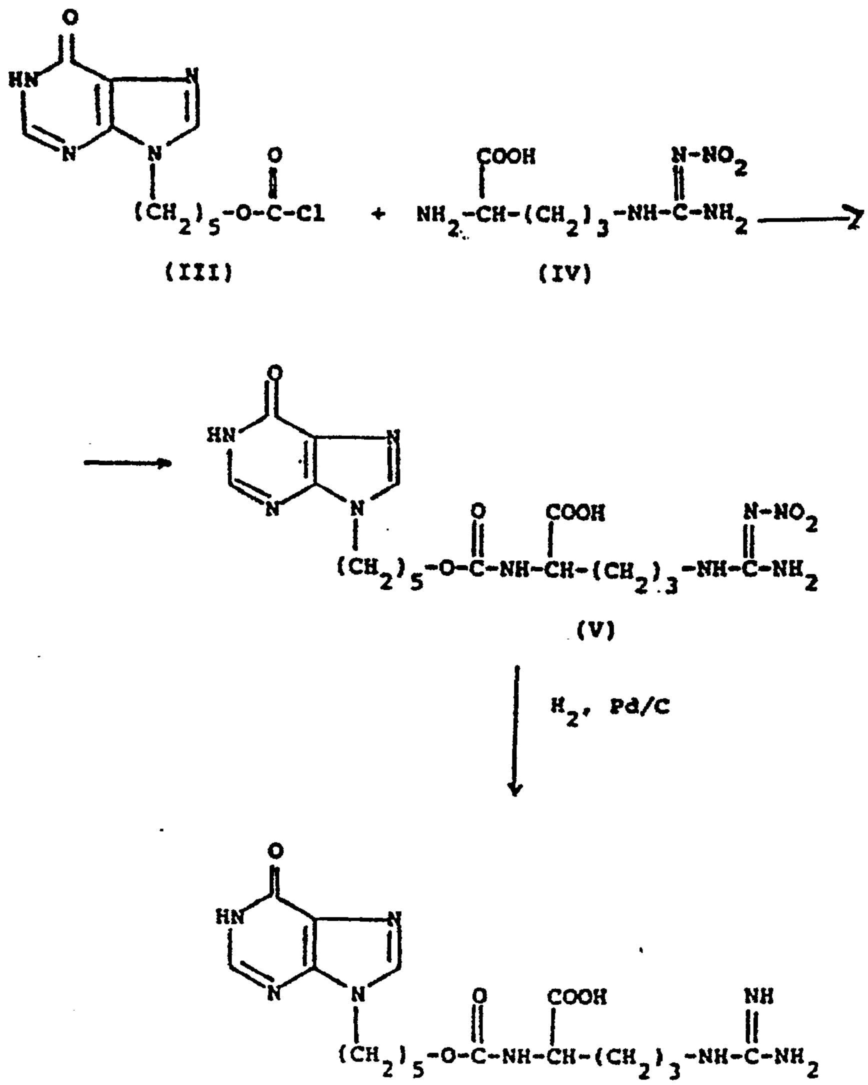


(III) (IV) (V)

(I)

2. Un procédé selon la revendication 1, caractérisé en ce que l'hydrogénolyse de V est effectuée sur du palladium sur charbon dans une solution alcoolique d'acide acétique.

3. Un procédé selon la revendication 2, caractérisé en ce que la réaction est conduite dans une solution méthanolique.

4. Un procédé selon la revendication 1, caractérisé en ce que l'acylation de IV avec III est effectuée dans un système hétérogène, constitué d'une solution aqueuse d'un sel IV et d'une suspension de III dans un solvant aprotique.

5. Un procédé selon la revendication 4, caractérisé en ce que le sel IV est le sel de sodium ou de potassium.

**Patentansprüche**

1. Verfahren zur Herstellung von α-N-[(Hypoxanthin-9-yl)-pentyloxycarbonyl]-arginin der Formel I, dadurch gekennzeichnet, dass α-N-[(Hypoxanthin-9-yl)-pentyloxycarbonyl]-$N_7$-itroarginin der Formel V, erhalten durch Acylierung von $N_7$-Nitroarginin IV mit (Hypoxanthin-9-yl)-pentyloxycarbonylchlorid III, der Hydrogenolyse unterzogen wird nach dem Schema

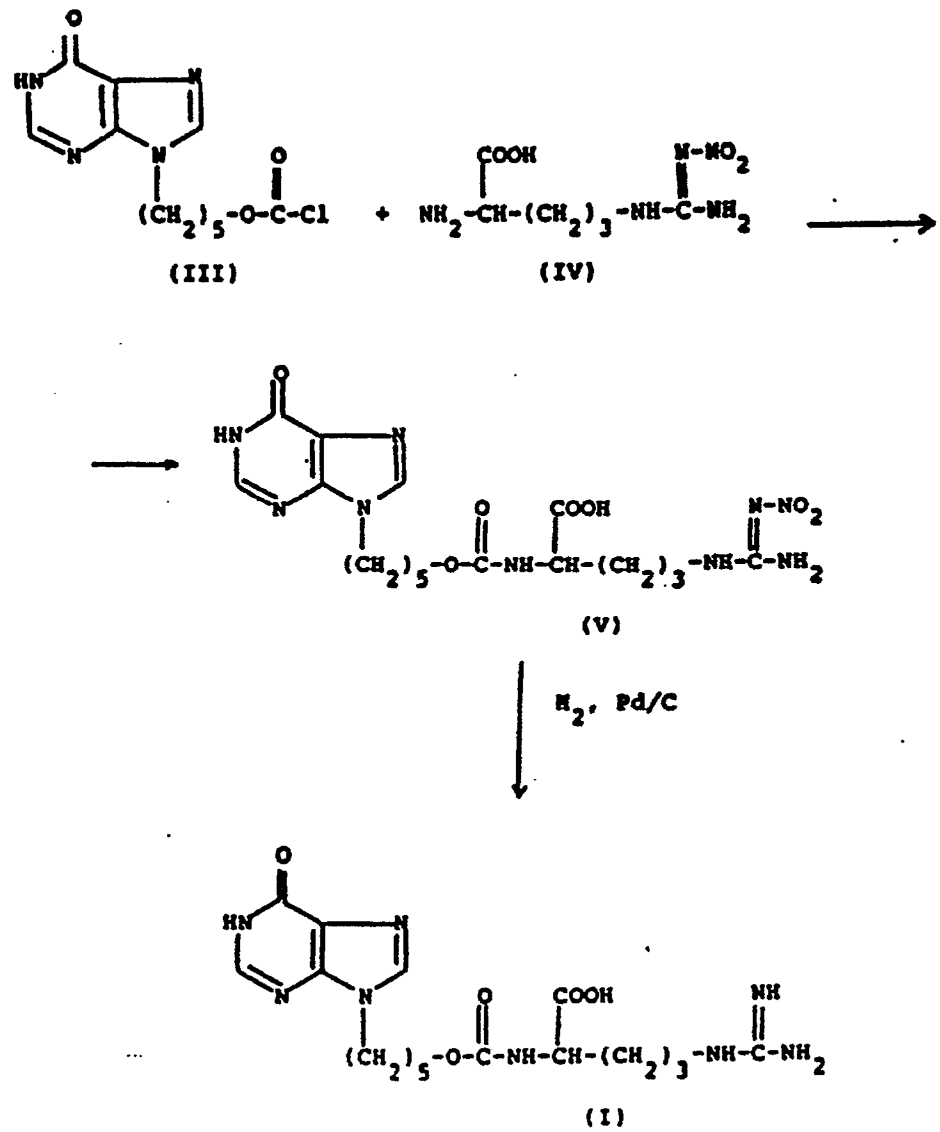


(III) (IV)

(V)

(I)

2: Verfahren nach Anspruch 1, dadurch gekennzeichnet, dass die Hydrogenolyse von V durchgeführt wird auf Palladium auf Aktivkohle in alkoholischer Essigsäurelösung.

3. Verfahren nach Anspruch 2, dadurch gekennzeichnet, daß die Reaktion in Methanollösung durchgeführt wird.

4. Verfahren nach Anspruch 1, dadurch gekennzeichnet, daß die Acylierung von IV mit III durchgeführt wird in einem heterogenen System, bestehend aus einer wässrigen Lösung eines Salzes IV und einer Suspension von III in einem aprotischen Lösungsmittel.

5. Verfahren nach Anspruch 4, dadurch gekennzeichnet, dass das Salz IV das Natrium- oder Kaliumsalz ist.